# EUROPEAN PATENT APPLICATION

(11) **EP 1 839 643 A1**
(43) Date of publication of application: **03.10.2007**
(21) Application number: 07104515.7
(22) Date of filing: 20.03.2007
(51) Int. Cl.: A61K 8/04, A61K 8/34, A61K 8/58, A61K 8/86, A61Q 19/00, A61K 47/10, A61K 47/44, A61P 17/00, A61P 17/04, A61P 17/10, A61K 31/16, A61K 31/4178, A61K 31/573, A61P 17/06

(54) **Cosmetic and pharmaceutical foam carrier**

(30) Priority: 21.03.2006 IL 17444706; 18.05.2006 IL 17573506
(71) Applicant: Kamedis Ltd., 61171 Tel Aviv (IL)
(72) Inventor: Konis, Yoel, 38001, Hadera (IL); Kalay, Amir, 69355, Tel Aviv (IL)
(74) Representative: Pohlman, Sandra M.

(57) **Abstract**

The invention provides a quick-break, alcohol-free, cosmetic and pharmaceutical foam carrier comprising about 20%-60% hydrophobic volatile solvent, about 20%-60% water, about 3%-20% of polyol, and about 0.1%-7.5% of a surface active agent.

## Description

The present invention relates to a quick-break, alcohol free cosmetic and pharmaceutical foam carrier and uses thereof.

More specifically, the present invention relates to a cosmetic or pharmaceutical foam carrier suited for inclusion in both water-soluble and oil-soluble cosmetic agents.

As already described in the background of WO 2004/037225, external topical administration is an important route for the administration of drugs in disease treatment. In external topical administration, the drug is absorbed into and/or through skin, mucous membrane or wound issue. Many groups of drugs, including, for example, antibiotic, anti-fungal, anti-inflammatory, anesthetic, analgesic, corticosteroid, retinoid and anti-proliferative medications are preferably administered in hydrophobic media, e.g. ointments or oils. However, due to the undesirable consistency of these hydrophobic carriers, their use is limited. For instance, ointments containing white petrolatum, e.g., Vaseline^{®} petroleum jelly, as the carrier often form an impermeable barrier, so that metabolic products and excreta from the wounds to which they are applied are not easily removed or drained away. Furthermore, it is difficult for the active drug dissolved in the carrier to pass through the white petrolatum barrier layer into the wound tissue, so the efficacy of the drug is reduced.

In addition, ointments and creams often do not create an environment for promoting respiration of the wound tissue and it is not favorable to the normal respiration of the skin. An additional disadvantage of petroleum jelly-based products relates to the greasy feeling left following their topical application onto the skin, mucosal membranes and wounds. Besides petroleum jelly, hydrophobic pharmaceutical carriers now in use include liquid paraffin, lanolin, beeswax, vegetable oil, and glycerin monostearate; and hydrophilic carriers include higher alcohols, polyethylene glycol and some emulsifying agents, which also have undesirable flow properties and skin feel.

Several hydrophobic liquid and semi-solid oils, e.g., mono- and polyunsaturated oils from vegetable and marine sources, mineral oils, silicone oils, and liquid hydrophobic plant-derived oils, are known for their therapeutic benefits when applied topically, yet, their application in liquid form is not practical. Oils can also contain essential nutritional constituents, such as oil-soluble vitamins (e.g., vitamin A, D and vitamin E), minerals and other therapeutically beneficial constituents. Another class of therapeutic oils includes mineral and silicon oils useful for the treatment of skin dehydration and other medical disorders, which oils are liquid at ambient temperature..

Other pharmaceutical active ingredients are water-soluble and require a water component in the carrier.

While semi-solid cosmetic and pharmaceutical formulations, such as creams, lotions, gels and ointments are commonly used by consumers, new forms are desirable, in order to achieve better control of the application, while maintaining or bestowing the skin beneficial properties of such products. Thus, the development of a new composition, having breakable foam consistency when extruded out of a container and liquid properties when applied onto the skin is advantageous. Ideally a foam should contain hydrophobic substances (solvents), which can act as emollients and provide the skin with soothing and nourishing properties. However, such hydrophobic solvents are difficult to formulate into a lather-producing or foam-producing product because the hydrophobic solvents interfere with the lather forming ability of the surfactant. Furthermore, addition of oils and other emollients to topical formulations can result in unpleasant or annoying skin residue.

Use of emulsions in foam compositions is known. Emulsion systems provide a two-phase system including lipophilic or hydrophobic components in one phase and hydrophilic components in the second phase. The foamed emulsion typically is an oil-in-water emulsion in which the hydrophobic component is dispersed in the aqueous continuous phase. Surfactants for reducing surface tension and emulsifiers for improving foam stability are included in the foam composition.

Foams, and in particular foam emulsions, are complicated systems which do not form under all circumstances. Slight shifts in foam emulsion composition, such as the addition of active ingredients, may destabilize the foam. Furthermore, many emulsions do not provide the high foam capacity, foam stability and/or fast-breaking action under stress or temperatures that are desired in a topical foam composition.

A particularly desirable type of oil-containing foam is such wherein all or part of the oil phase comprises silicone oil. Silicone oil is known for its skin protective features and its incorporation in topical products is beneficial.

However, it is not obvious to produce silicone oil-based foams, since many silicone oils possess anti-foaming properties.

US Patent No. 6,126,920 discloses treatment of various skin diseases, and in particular, scalp psoriasis, using a foamable pharmaceutical composition containing a corticosteroid active substance, an aliphatic alcohol, water, a fatty alcohol, a surface-active agent, a propellant and a buffering agent. The foamable composition contains 40-90% w/w composition of an aliphatic alcohol. US Pat. No. 6,126,920 is typical of many compositions that use aliphatic alcohols in the foam composition. The alcohol promotes fast drying and thereby attempts to address the sticky feeling left by many topical formulations after application; however, alcohols, and in particular the methyl, ethyl and isopropyl alcohols preferred in the '920 patent, are defatting agents and may cause skin to become dry and cracked. Hence, the presence of aliphatic alcohol in a therapeutic foam for external topical administration as taught in US Patent No. 6,126,920 is undesirable.

US Patent No. 5,536,743 to Borgman describes a buffered non-flowing composition suitable for the treatment of bacterial vaginosis, which contains metronidazole. Suitable formulations include oil-in-water emulsions including an internal oil phase of about 10-40 wt% oil and anionic, cationic or nonionic surfactants. Suitable components of the oleaginous phase include long chain alcohols, esters, and acids, vegetable and animal oils and waxes. No other stabilizing agents are disclosed for use in foam aerosol compositions.

EP 0,598,412 describes a composition that is useful for skin protection against drying and harsh environmental substances. The protection is derived from the inclusion of poly(tetrafluoroethylene) (PTFE) in the composition. The composition includes low levels of both hydrophilic emollients and hydrophobic emollients. The compositions include high levels of surfactants, including ionic surfactants, and co-emulsifiers, resulting in thick emulsions which are not flowable, and thus provide products which are inefficient foamers (or non-foaming) and too thick for spreading over large skin areas.

US Patent 6,423,323 describes an aqueous foam emulsion. The composition includes a hydrophobic phase including fatty acids, emulsifiers and co-emulsifiers, and an aqueous phase containing hydrophilic moisturizers and emulsifiers. An optional ingredient according to US Pat. 6,423,323 is one or more refatting substances, in preferable concentrations of 0.5 to 2%, if the product is to be used for normal skin; and 3 to 6% for dry skin. Addition of high levels of co-emulsifiers such as fatty alcohols and fatty acids suggest that the foam is not stable. No other stabilizing agents are disclosed.

US Patent 5,635,469 describes a foamable cleansing liquid composition comprising about 0.05% to about 10% of an emollient, in addition to cleansing surfactants, humectants and water soluble cationic or nonionic polymers, but no propellants. Low density foams are achieved using a novel non-aerosol foam dispenser. The foaming is achieved by operating a manual pump, which is not convenient for operation. Emollients and humectants are included to improve the level of hydration and/or lipid content of the skin. However, the patent notes that emollients and humectants interfere with the lather forming ability of the surfactant.

US Patent 6,113,888 teaches a single water phase composition comprising a self-tanning agent, a nitrogen-free polymer, a nitrogen-free surfactant, and water and therefore does not teach or suggest the composition of the present invention.

US Pat. 5,679,324 to Lisboa, pertains to an aerosol foamable fragrance composition, translucent in its pre-dispensed state, which forms a fast breaking foam. Apparently the foam breaks spontaneously upon discharging from an aerosol container (with no need of any rubbing or sheer force application), thus, making it impractical for spreading over a skin surface. The composition contains surfactant, a propellant, a fragrance, a thickener, and a cosmetic vehicle (preferably water) wherein the ratio of the surfactant to propellant is from about 1:1 to about 1:10. Emollients including silicone oils, mineral oils and hydrocarbon oils may be included.

US Patent 6,251,369 discloses foamable dental fluoride compositions containing a water-soluble fluoride component, whereby said compositions include an oil in water emulsion. However, the patent fails to specify the identity or concentration of the oil component of the emulsion; and none of the compositions presented in the examples contain any oil component.

US Patent 5,961,957 describes a barrier foam composition comprising from 70 to 90% of water, from 7 to 9% of butane, from 2 to 4% of glyceryl monostearate, from 1.5 to 3.50% of dimethicone copolyol (a water-soluble silicone compound), from 1 to 3% of propane, from 0.5 to 2.5% of lanolin, from 0.5 to 2.5% of stearic acid and from 0.05 to 1.05% of at least one of methylchloroisothiazolinone and methylisothiazolinone.

Israel Specification No. 152,486 and in W02004/037225 to Foamix disclose the use of an alcohol free foamable carrier composed of 2 - 75% fluid non-volatile hydrophobic solvent, plus 80 - 88% water, plus 0.1 - 5% adjuvant agent selected from fatty alcohols, fatty acids plus 0.1% - 5% surface active agent and .01% - 5% water gelling agent which is a mast for stabilizing the foam. The final formula also contains a propellant. In this invention the water gelling agent is a must for the foam to retain its structure for a time sufficient for the user to apply and rub the foam into the skin. The inclusion of any water gelling agent in the composition makes the foam sticky upon drying and decreases its vanishing speed. The stickiness and low vanishing speed makes the foam less accepted by the end user.

A few dermatological foam products are available on the market.
Olux^{™} Foam, produced by Connetics, Inc., contains clobetasol propionate. Each gram of Olux^{™} Foam contains 0.5 mg clobetasol propionate, USP, in a thermolabile foam, which consists of ethanol (60%), purified water, propylene glycol, cetyl alcohol, stearyl alcohol, polysorbate 60, citric acid, and potassium citrate. It is dispensed from an aluminum can pressurized with a hydrocarbon propellant (propane/butane). Luxiq ^{™} is another corticosteroid foam medication, containing 1.2 mg betamethasone valerate per gram, in a vehicle, comprising ethanol (60.4%), purified water, propylene glycol, cetyl alcohol, stearyl alcohol, polysorbate 60, citric acid, and potassium citrate, and pressurized with a hydrocarbon propellant.

Cortifoam^{®}, a hydrocortisone acetate rectal foam is produced by Schwartz Pharma GmbH, wherein the hydrocortisone is present at 1 0% in a foam vehicle. Nonmedicinal ingredients of Cortifoam^{®}, include cetyl alcohol, ethoxylated stearyl alcohol, methylparaben, polyoxyethylene-1 0 stearyl ether, propylene glycol, propylparaben, triethanolamine, water, and inert propellants, isobutene, and propane.

Thus, quick-break foam compositions for topical treatment, containing higher concentrations of oils, and that do not comprise alcohol are still desirable. Foam compositions that are robust and suitable for the inclusion of a wide range of active ingredients are desired.

Thus according to the present invention there is now provided a quick-break, alcohol-free, cosmetic and pharmaceutical foam carrier comprising about 20%-60% hydrophobic volatile solvent, about 20%-60% water, about 3%-20% of polyol, and about 0.1%-7.5% of a surface active agent.

A further embodiment provided by the present invention is a quick-break, alcohol-free, cosmetic and pharmaceutical foam carrier, comprising about 30%-50% hydrophobic volatile solvent, about 20%-40% water, about 3%-20% of a polyol, and about 0.1 %-7.5% of a surface active agent and about 0.1 %-5% of a foam adjuvant agent.

A preferred embodiment provided by the present invention is a quick-break, alcohol-free, cosmetic and pharmaceutical foam carrier, comprising about 30%-50% hydrophobic volatile solvent, about 20%-40% water, about 3%-20% of a polyol, about 0.1%-7.5% of a surface active agent, about 0.1%-5% of a foam adjuvant agent, about 0.1 %-3% of a non-volatile hydrophobic compound.

In yet another embodiment of the present invention there is now provided a quick-break, alcohol-free, cosmetic and pharmaceutical foam carrier wherein said non-volatile hydrophobic compound possesses initial foam stability, solubility in aqueous / volatile oil solutions and dry lubricity.

In a preferred embodiment of the present invention there is now provided a quick-break, alcohol-free, cosmetic and pharmaceutical foam carrier wherein said polyols are selected from those having skin moisturizing properties, drug and cosmetic penetration enhancing properties and active material solubilizer properties.

Preferred hydrophobic volatile solvents for use in the present invention are selected from the group consisting of: Disiloxane (Hexamethyldisiloxane), Dimethicone (0.65 CST-Volatile) and Cyclomethicone (C₄ or C₅).

Polyols that are preferred for use in the present invention are selected from the group consisting of: PEG-12 Dimethicone, Hexylene Glycol, Propylene Glycol, Butylene Glycol, Ethoxydiglycol and Glycerin.

Preferred foam adjuvants for use in the present invention are selected from the group consisting of: Cetearyl Alcohol and Stearyl Alcohol.

The preferred surface active agents for use in the present invention are selected from the group consisting of: Oleth-20, Ceteareth-20, and Laneth-40.

The preferred non-volatile hydrophobic compounds possessing initial foam stability and solubility in aqueous/volatile oil solutions (with dry lubricity) for use in the present invention are selected from the group consisting of: Diisopropyl Adipate, Propylene Carbonate, Dimethyl Sebacate and Diethyl Succinate.

In preferred embodiments of the present invention said foam compositions are further combined with a suitable Propellant. Propellants that are preferred for use in the present invention are selected from the group consisting of Dimethylether, Isobutane and a Propane Butane mixture.

When the carriers of the present invention are used as quick-break, alcohol-free pharmaceutical foam carriers for external, topical drug formulations and even for veterinary topical formulations, the active ingredient is preferable selected from the group consisting of corticosteroids, fungicides and antibiotics.

More specifically said corticosteroids are preferably selected from the group consisting of Hydrocortisone, Dexamethasone, Pretnisolone and Betamethasone, said antifungal agents are preferably selected from the group consisting of Climbazol, Clotrimazole, Ketokonasole and Bifonazole, and said antibiotics are preferably selected from the group consisting of Chloramphenicol and Tretracycline.

Topical pharmaceutical and veterinary compositions formed according to the present invention can also contain more than one active ingredient.

While the invention will now be described in connection with certain preferred embodiments in the following examples so that aspects thereof may be more fully understood and appreciated, it is not intended to limit the invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

### Example 1

A quick-break, alcohol-free, cosmetic foam carrier was prepared having the following formulation:

| | | | % |
|---|---|---|---|
| | Disiloxane (0.65 CST-Volatile) | | 43.00 |
| | Glycerin | | 5.00 |
| | Oleth-20 | } | 5.00 |
| | Water | | up to 100 |
| Active Ingredients: | Dipotassium Glycyrrhizate | | 1.00 |
| | Acetyl Hexapeptide-1 | | 2.00 |
| Preservative: | Sodium Methylparaben | | 0.25 |
| Fragrance: | Rose Water | | 0.10 |
| Propellant: | Dimethylether | | 6.80 |

### Example 2

A quick-break, alcohol-free, cosmetic foam carrier was prepared having the following formulation:

| | | % |
|---|---|---|
| | Cyclomethicone | 52.00 |
| | Cetearyl Alcohol & } | |
| | Ceteareth-20 } | 3.5 |
| | Laneth-40 | 1.50 |
| | Propylene glycol | 5.00 |
| | Water | up to 100 |
| Active Ingredients: | Taraktogenos Kurgii Seed Oil | 1.70 |
| | Sophora Augustifolia Extract | 1.70 |
| | Cnidium Monnieri Extract | 1.70 |
| Preservative : | Sodium Methylparaben | 0.20 |
| Fragrance: | Rose Water | 0.10 |
| Propellant: | Dimethylether | 5.00 |

### Example 3

A quick-break, alcohol-free, cosmetic foam carrier was prepared having the following formulation:

| | | % |
|---|---|---|
| | Dimethicone (0.65 CST-Volatile) | 43.70 |
| | Oleth-20 | 2.50 |
| | Cetearyl Alcohol & } | |
| | Ceteareth-20 } | 2.50 |
| | Butylene Glycol | 5.00 |
| | Hexylene Glycol | 5.00 |
| | Diisopropyl Adipate | 1.00 |
| | Water | up to 100 |
| Active Ingredient: | Borneol | 0.09 |
| Preservative: | Sodium Methylparaben | 0.40 |
| Propellant: | Isobutane | 8.00 |

### Example 4

A quick-break, alcohol-free, cosmetic foam carrier was prepared having the following formulation:

| | | % |
|---|---|---|
| | Dimethicone (0.65 CST-Volatile) | 44.00 |
| | Oleth-20 | 2.50 |
| | Cetearyl Alcohol & } | |
| | Ceteareth-20 } | 2.50 |
| | Propylene Glycol | 10.00 |
| | Ethoxydiglycol | 1.00 |
| | Propylene Carbonate | 1.00 |
| | Water | up to 100 |
| Active Ingredients: | Caprylic/Capric Triglyceride & } | |
| | Laminaria Ochroleuca Extract} | 2.00 |
| Preservative: | Imidazolidinyl Urea | 0.30 |
| Propellant: | Dimethylether | 6.50 |

### Example 5

A quick-break, alcohol-free, cosmetic foam carrier was prepared having the following formulation:

| | | % |
|---|---|---|
| | Disiloxane (0.65 CST-Volatile) | 48.17 |
| | Oleth-20 | 2.50 |
| | Cetearyl Alcohol & } | |
| | Ceteareth-20 } | 2.50 |
| | Glycerin | 5.00 |
| | Hexylene Glycol | 5.00 |
| | Diisopropyl Adipate | 1.00 |
| | PEG-12 Dimethicone | 1.00 |
| | Water | up to 100 |
| Active Ingredient: | Borneol | 0.09 |
| Preservative: | Sodium Methylparaben | 0.20 |
| Propellant: | Dimethylether | 6.80 |

### Example 6

A quick-break, alcohol-free, cosmetic foam carrier was prepared having the following formulation:

| | | % |
|---|---|---|
| | Dimethicone (0.65 CST-Volatile) | 43.70 |
| | Oleth-20 | 2.50 |
| | Cetearyl Alcohol & } | |
| | Ceteareth-20 } | 2.50 |
| | Butylene Glycol | 5.00 |
| | Hexylene Glycol | 5.00 |
| | Diisopropyl Adipate | 1.00 |
| | Water | up to 100 |
| Active Ingredient: | Borneol | 0.09 |
| Active Pharmaceutical | | |
| Ingredient: | Hydrocortisone | 2.00 |
| Preservative: | Sodium Methylparaben | 0.40 |
| Propellant: | Dimethylether | 6.50 |

### Example 7

A quick-break, alcohol-free, cosmetic foam carrier was prepared having the following formulation:

| | | % |
|---|---|---|
| | Dimethicone (0.65 CST-Volatile) | 42.50 |
| | Hexylene Glycol | 10.00 |
| | Ethoxydiglycol | 2.00 |
| | Oleth-20 | 3.00 |
| | Cetearyl Alcohol & } | |
| | Ceteareth-20 } | 2.00 |
| | Water | up to 100 |
| Active Ingredient: | Acetyl Hexapeptide-1 | 4.00 |
| Preservative: | Sodium Methylparaben | 0.30 |
| Propellant: | Dimethylether | 5.00 |

### Example 8

A quick-break, alcohol-free, cosmetic foam carrier was prepared having the following formulation:

| | | % |
|---|---|---|
| | Dimethicone (0.65 CST-Volatile) | 49.00 |
| | Cetearyl Alcohol & } | |
| | Ceteareth-20 } | 2.00 |
| | Oleth-20 | 4.50 |
| | Propylene Glycol | 10.00 |
| | Water | up to 100 |
| Active Ingredients: | Acetyl Hexapeptide-1 | 4.00 |
| | Cnidium Monnieri Extract | 3.40 |
| | Dipotassium Glycyrrhizate | 2.00 |
| Preservative: | Sodium Methylparaben | 0.25 |
| Propellant: | Dimethylether | 6.00 |

### Example 9

A quick-break, alcohol-free, cosmetic foam carrier was prepared having the following formulation:

| | | % |
|---|---|---|
| | Disiloxane (0.65 CST-Volatile) | 47.00 |
| | Oleth-20 | 5.00 |
| | Propylene Glycol | 10.00 |
| | Diisopropyl Adipate | 1.00 |
| | PEG-12 Dimethicone | 1.00 |
| | Water | up to 100 |
| Active Ingredients: | Caprylic/Capric Triglyceride & } | |
| | Laminaria Ochroleuca Extract} | 2.00 |
| Preservative: | Sodium Methylparaben | 0.20 |
| Propellant: | Dimethylether | 6.80 |

### Example 10

A quick-break, alcohol-free, cosmetic foam carrier was prepared having the following formulation:

| | | % |
|---|---|---|
| | Dimethicone (0.65 CST-Volatile) | 46.80 |
| | Oleth-20 | 3.50 |
| | Ceteareth-20 | 1.50 |
| | Glycerin | 5.00 |
| | Hexylene Glycol | 5.00 |
| | Diisopropyl Adipate | 1.00 |
| | Water | up to 100 |
| Active Ingredients: | Taraktogenos Kurzii Seed Oil | 1.65 |
| | Sophora Angustifolia Extract | 1.65 |
| | Cnidium Monnieri Extract | 1.65 |
| Preservative: | Sodium Methylparaben | 0.20 |
| Propellant: | Dimethylether | 6.80 |

### Example 11

A quick-break, alcohol-free, pharmaceutical foam carrier was prepared having the following formulation:

| | | % |
|---|---|---|
| | Cyclomethicone | 27.50 |
| | Cetearyl Alcohol & } | |
| | Ceteareth-20 } | 3.50 |
| | Water | up to 100 |
| Active Ingredients: | Hydrocortisone | 1.00 |
| | Tetracycline | 1.00 |
| Preservative: | Sodium Methylparaben | 0.25 |
| Propellant: | Isobutane | 8.00 |

### Example 12

A quick-break, alcohol-free, pharmaceutical foam carrier was prepared having the following formulation:

| | | % |
|---|---|---|
| | Cyclomethicone | 52.00 |
| | Cetearyl Alcohol & } | |
| | Ceteareth-20 } | 3.50 |
| | Laneth-40 | 1.50 |
| | Water | up to 100 |
| Active Ingredients: | Clotrimazole | 1.50 |
| Preservative: | Sodium Methylparaben | 0.20 |
| Propellant: | Dimethyl Ether | 5.00 |

### Example 13

A quick-break, alcohol-free, pharmaceutical foam carrier was prepared having the following formulation:

| | | % |
|---|---|---|
| Dimethicone (0.65 CST-Volatile) | | 43.70 |
| Oleth-20 | | 2.50 |
| | Ceteareth-20 & } | |
| | Cetearyl Alcohol } | 2.50 |
| | Butylene Glycol | 5.00 |
| | Hexylene Glycol | 5.00 |
| | Diisopropyl Adipate | 1.00 |
| | Water | up to 100 |
| Active Ingredients: | Dexamethasone | 0.29 |
| Preservative: | Sodium Methylparaben | 0.40 |
| Propellant: | Dimethyl Ether | 6.50 |

### Example 14

A quick-break, alcohol-free, pharmaceutical foam carrier was prepared having the following formulation:

| | | % |
|---|---|---|
| | Dimethicone (0.65 CST-Volatile) | 44.00 |
| | Oleth-20 | 2.50 |
| | Ceteareth-20 & } | |
| | Cetearyl Alcohol } | 2.50 |
| | Propylene Glycol | 10.00 |
| | Ethoxydiglycol | 2.00 |
| | Propylene Carbonate | 1.00 |
| | Water | up to 100 |
| Active Ingredients: | Ketokonasole | 1.50 |
| Preservative: | Imidazolidinyl Urea | 0.30 |
| Propellant: | Dimethyl Ether | 6.50 |

### Example 15

A quick-break, alcohol-free, pharmaceutical foam carrier was prepared having the following formulation:

| | | % |
|---|---|---|
| Disiloxane (0.65 CST-Volatile) | | 48.17 |
| Oleth-20 | | 2.50 |
| Ceteareth-20 & } | | |
| Cetearyl Alcohol } | | 2.50 |
| | Glycerin | 5.00 |
| | Hexylene Glycol | 5.00 |
| | Diisopropyl Adipate | 1.00 |
| | PEG -12 Dimethicone | 1.00 |
| | Water | up to 100 |
| Active Ingredients: | Hydrocortisone | 2.00 |
| Preservative: | Sodium Methylparaben | 0.20 |
| Propellant: | Dimethyl Ether | 6.80 |

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A quick-break, alcohol-free, cosmetic and pharmaceutical foam carrier comprising about 20%-60% hydrophobic volatile solvent, about 20%-60% water, about 3%-20% of polyol, and about 0.1%-7.5% of a surface active agent.

2. A quick-break, alcohol-free, cosmetic and pharmaceutical foam carrier according to claim 1, comprising about 30%-50% hydrophobic volatile solvent, about 20%-40% water, about 3%-20% of polyol, and about 0.1 %-7.5% of a surface active agent and about 0.1 %-5% of foam adjuvant agent.

3. A quick-break, alcohol-free, cosmetic and pharmaceutical foam carrier according to claim 2, comprising about 30%-50% hydrophobic volatile solvent, about 20%-40% water, about 3%-20% of a polyol and about 0.1 %-7.5% of a surface active agent, about 0.1%-5% of a foam adjuvant agent and about 0.1 %-3% of a non-volatile hydrophobic compound.

4. A quick-break, alcohol-free, cosmetic and pharmaceutical foam carrier according to claim 3 wherein said non-volatile hydrophobic compound possesses initial foam stability, solubility in aqueous / volatile oil solutions and dry lubricity.

5. A quick-break, alcohol-free, cosmetic and pharmaceutical foam carrier according to claim 1 wherein said polyols are selected from those having skin moisturizing properties, drug and cosmetic penetration enhancing properties and active material solubilizer properties.

6. A quick-break, alcohol-free, cosmetic and pharmaceutical foam carrier according to claim 1 wherein said hydrophobic volatile solvents are selected from the group consisting of: Disiloxane (Hexamethyldisiloxane), Dimethicone (0.65 CST-Volatile) and Cyclomethicone (C₄ or C₅).

7. A quick-break, alcohol-free, cosmetic and pharmaceutical foam carrier according to claim 3 wherein said foam adjuvant agent is selected from the group consisting of Cetearyl Alcohol and Stearyl Alcohol.

8. A quick-break, alcohol-free, cosmetic and pharmaceutical foam carrier according to claim 1 wherein said Polyols are selected from the group consisting of: PEG-12 Dimethicone, Hexylene Glycol, Propylene Glycol, Butylene Glycol, Ethoxydiglycol and Glycerin.

9. A quick-break, alcohol-free, cosmetic and pharmaceutical foam carrier according to claim 1 wherein said surface active agent is selected from the group consisting of: Ceteareth-20, Oleth-20 and Laneth-40.

10. A quick-break, alcohol-free, cosmetic and pharmaceutical foam carrier according to claim 3 wherein said non-volatile hydrophobic compound, possesses initial foam stability and solubility in aqueous/volatile oil solutions and is selected from the group consisting of: Diisopropyl Adipate, Propylene Carbonate, Dimethyl Sebacate and Diethyl Succinate.

11. A quick-break, alcohol-free, cosmetic and pharmaceutical foam carrier according to claim 1 further comprising a Propellant selected from the group consisting of Dimethylether, Isobutane and a Propane Butane mixture.
